(19) Europäisches Patentamt
European Patent Office
Office européen des brevets

(11) **EP 4 506 022 A1**

(12) **EUROPEAN PATENT APPLICATION**

(43) Date of publication:
**12.02.2025 Bulletin 2025/07**

(21) Application number: **24193591.5**

(22) Date of filing: **08.08.2024**

(51) International Patent Classification (IPC):
**A61M 1/02** *(2006.01)*    **A61M 1/36** *(2006.01)*

(52) Cooperative Patent Classification (CPC):
**A61M 1/0272; A61M 1/0209; A61M 1/362266;
A61M 1/3696;** A61M 1/36224; A61M 1/36225;
A61M 2202/0427; A61M 2202/0429;
A61M 2205/3306

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **11.08.2023   US 202363519100 P**

(71) Applicant: **Fenwal, Inc.
Lake Zurich, IL 60047 (US)**

(72) Inventors:
• **KUSTERS, Benjamin E.
Lake Zurich, 60047 (US)**
• **MIN, Kyungyoon
Lake Zurich, 60047 (US)**

(74) Representative: **Maikowski & Ninnemann
Patentanwälte Partnerschaft mbB
Postfach 15 09 20
10671 Berlin (DE)**

(54) **METHOD FOR DETERMINING RED CELL ADDITIVE SOLUTION VOLUME**

(57)    Automated blood processing systems and methods including a durable hardware component, a single use fluid flow circuit, and a controller configured to determine and add a custom amount of additive solution. More particularly, systems and methods for adding a custom amount of a red blood cell (RBC) additive solution to obtain a target RBC product with a target hematocrit.

FIG. 1

EP 4 506 022 A1

## Description

Field of Disclosure

The present disclosure relates generally to systems, devices, and methods for processing blood. More particularly, the disclosure relates generally to systems, devices, and methods for separating and collecting blood components with a particular collection parameter. Additionally, the disclosure relates to systems, devices, and methods of determining and adding a custom amount of additive solution to a separated blood component.

Background

It is well known to collect whole blood from donors using manual collection procedures through blood drives, donor visits to blood centers or hospitals and the like. In such procedures, blood is typically collected by simply flowing it from the donor under the force of gravity and venous pressure into a collection container (e.g., a flexible pouch or bag), although various blood collection instruments may be used to aid or expedite the collection of blood or blood components.

The collection container in manual collection is often part of a larger preassembled arrangement of tubing and containers (sometimes called satellite containers) that are used in further processing of the collected whole blood. More specifically, the whole blood is typically first collected in what is called a primary collection container that also contains an anticoagulant, such as but not limited to a solution of sodium citrate, phosphate, and dextrose ("CPD").

After initial collection, it is a common practice to transport the collected whole blood to another facility or location, sometimes called a "back lab," for further processing to separate red blood cells ("RBCs"), platelet, and plasma from the whole blood, which may include carrying out additional processes, such as cell washing and plasma cryoprecipitate production and collection. This processing usually entails manually loading the primary collection container and associated tubing and satellite containers into a centrifuge to separate the whole blood into concentrated red cells and platelet-rich or platelet-poor plasma. The separated components may then be expressed from the primary collection container into one or more of the satellite containers, with the RBCs being combined with an additive or preservative solution pre-filled in one of the satellite containers. After the above steps, the blood components may be again centrifuged, if desired, for example to separate platelets from plasma. The overall process requires multiple large floor centrifuges and fluid expression devices. Because of the multiple operator interactions, the process is labor intensive, time consuming, and subject to human error.

When the desired product is RBCs, under current practices, a pre-determined volume of additive solution, for example 110 mL, is typically added to RBC products. This leads to red blood cell products with varying hematocrits based on the absolute volume of pure red blood cells supplied by a unit of blood. In some instances, it may be desirable to standardize the hematocrit of RBC products by adding different volumes of additive solution depending on the volume of pure RBCs supplied by a unit of whole blood.

Additionally, using current floor centrifuges and fluid expression for whole blood unit separation does not provide a reasonable opportunity to determine the volume of RBCs or to precisely add a specific volume of additive solution. The volume of pure RBCs in a unit of whole blood is dependent on the hematocrit of the donation, which is often unknown, and the volume of the whole blood unit. Accordingly, a fixed or pre-determined volume of additive solution is added to RBCs to simplify the process, which may lead to different RBC product hematocrits.

Therefore, there exists a need for devices, systems, and methods to automate and customize blood processing procedures and, for example, provide target blood component products with a target collection parameter.

Summary

There are several aspects of the present subject matter which may be embodied separately or together in the devices, systems, and methods described and/or claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto or later amended. For purposes of this description and claims, unless otherwise expressly indicated, "blood" is intended to include whole blood and blood components, such as concentrated red cells, plasma, platelets and white cells, whether with or without anticoagulant or additives.

In one aspect, a system for collecting a blood component is provided. The system includes a durable hardware component. The durable hardware component includes a separator and a controller. The controller is configured to automatically operate the system to separate a target blood component from blood withdrawn from a blood source and determine and add a custom amount of additive solution to the target blood component to obtain a blood component product with a target collection parameter. The system also includes a disposable fluid flow circuit for mounting onto the durable hardware component. The disposable fluid flow circuit includes a separation chamber and a plurality of containers in fluid communication with the separation chamber. The plurality of containers includes at least a product container configured to contain the blood component product and an additive solution container configured to contain an additive solution.

In another aspect, a method of obtaining a separated blood component product is provided. The method includes separating and collecting a blood component from a blood source using a blood processing system. The blood processing system includes a durable hardware component including a separator and a controller. The controller is configured to automatically operate the system to separate a target blood component from a blood source and determine and add a custom amount of additive solution to the target blood component to obtain a blood component product with a target collection parameter. The system also includes a disposable fluid flow circuit for mounting onto the durable hardware component. The disposable fluid flow circuit includes a separation chamber and a plurality of containers in fluid communication with the separation chamber, wherein the plurality of containers includes at least a product container configured to contain the blood component product and an additive solution container configured to contain an additive solution. The method also includes adding the custom amount of additive solution to the blood component.

In yet another aspect, a blood processing device for collecting a separated blood component product is provided. The device includes a separator and a controller. The controller is configured to automatically operate the device to separate a target blood component from a blood source and determine and add a custom amount of additive solution to the target blood component to obtain a blood component product with a target collection parameter. The device is configured to be associated with a disposable fluid circuit.

These and other aspects of the present subject are set forth in the following detailed description of the accompanying drawings.

Brief Description of the Drawings

FIG. 1 is a perspective view of an exemplary reusable hardware component of a blood processing system which is configured to receive a disposable fluid flow circuit.

FIG. 2 is a plan view of an exemplary disposable fluid flow circuit for use in combination with the durable hardware component of FIG. 1.

FIG. 3 is a schematic view of the fluid flow circuit of FIG. 2 mounted to the processing device of FIG. 1 to complete a blood processing system according to an aspect of the present disclosure.

FIG. 4 is a schematic view of another embodiment of a fluid flow circuit mounted to the processing device of FIG. 1 to complete a blood processing system according to an aspect of the present disclosure.

Detailed Description of the Embodiments

The embodiments disclosed herein are for the purpose of providing an exemplary description of the present subject matter. They are, however, only exemplary and not exclusive, and the present subject matter may be embodied in various forms. Therefore, specific details disclosed herein are not to be interpreted as limiting the subject matter as defined in the accompanying claims.

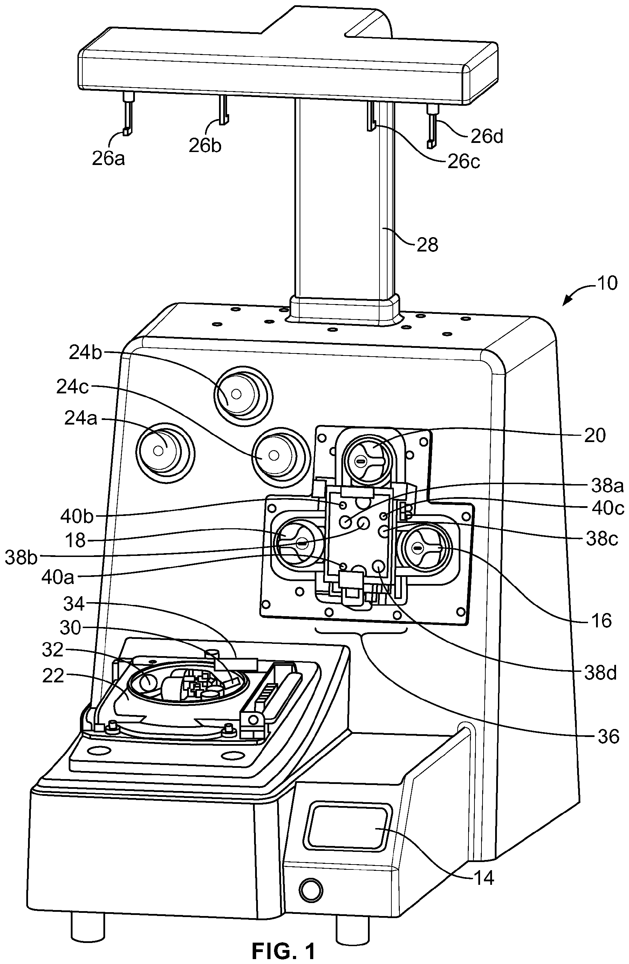

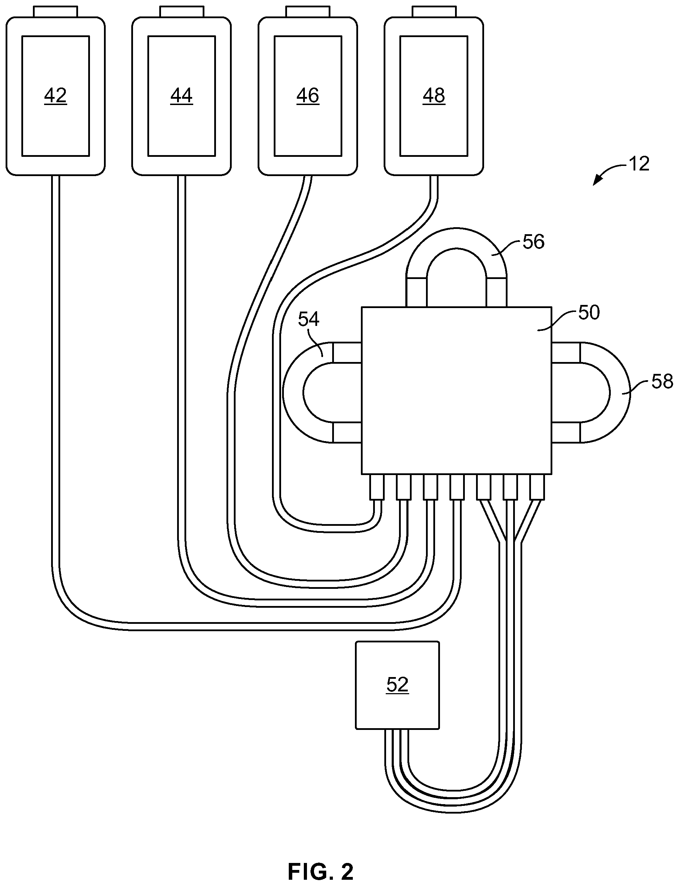

FIG. 1 depicts a reusable or durable hardware component or processing device 10 of a configurable, automated blood processing system or blood component manufacturing system, while FIG. 2 depicts a disposable or single use fluid flow circuit 12 to be used in combination with the processing device 10 for processing collected whole blood. The illustrated processing device 10 includes associated pumps, valves, sensors, displays, and other apparatus for configuring and controlling flow of fluid through the fluid flow circuit 12, described in greater detail below. The blood processing system may be directed by a controller integral with the processing device 10 that includes a programmable microprocessor to automatically control the operation of the pumps, valves, sensors, etc. The processing device 10 may also include wireless communication capabilities to enable the transfer of data from the processing device 10 to the quality management systems of the operator. The processing device 10 may be the processing device described in WO 2021/194824, filed March 17, 2021, the disclosure of which is hereby incorporated by reference herein in its entirety.

More specifically, the illustrated processing device 10 includes a user input and output touchscreen 14, a pump station or system including a first pump 16 (for pumping, e.g., whole blood), a second pump 18 (for pumping, e.g., plasma) and a third pump 20 (for pumping, e.g., additive solution), a separator 22 (which may be, for example, a centrifuge), and clamps 24a-c. The touchscreen 14 enables user interaction with the processing device 10, as well as the monitoring of procedure parameters, such as flow rates, container weights, pressures, etc. The pumps 16, 18, and 20 (collectively referred to herein as being part of a "pump system" of the processing device 10) are illustrated as peristaltic pumps capable of receiving tubing or conduits and moving fluid at various rates through the associated conduit dependent upon the procedure being performed. An exemplary centrifuge mounting station/drive unit is seen in U.S. Patent No. 8,075,468 (with reference to Figs. 26-28), which is hereby incorporated herein by reference. The clamps 24a-c (collectively referred to herein as being part of the "valve system" of the processing device 10) are capable of opening and closing fluid paths through the tubing or conduits and may incorporate RF sealers in order to complete a heat seal of the tubing or conduit placed in the clamp to seal the tubing or conduit leading to a product container upon completion of a procedure.

Sterile connection/docking devices may also be incorporated into one or more of the clamps 24a-c. The sterile connection devices may employ any of several different operating principles. For example, known sterile connection devices and systems include radiant energy systems that melt facing membranes of fluid flow conduits, as in U.S. Patent No. 4,157,723; heated wafer systems that employ wafers for cutting and heat bonding or splicing tubing segments together while the ends remain molten or semi-molten, such as in U.S. Patent Nos. 4,753,697; 5,158,630; and 5,156,701; and systems employing removable closure films or webs sealed to the ends of tubing segments as described, for example, in U.S. Patent No. 10,307,582. Alternatively, sterile connections may be formed by compressing or pinching a sealed tubing segment, heating and severing the sealed end, and joining the tubing to a similarly treated tubing segment as in, for example, U.S. Patent Nos. 10,040,247 and 9,440,396. All of the above-identified patents are incorporated by reference in their entirety. Sterile connection devices based on other operating principles may also be employed without departing from the scope of the present disclosure.

The processing device 10 may also include hangers 26a-d (which may each be associated with a weight scale) for suspending the various containers of the disposable fluid circuit 12. The hangers 26a-d are preferably mounted to a support 28, which is vertically translatable to improve the transportability of the processing device 10. An optical system comprising a light source 30 and a photodetector 32 is associated with the separator 22 for determining and controlling the location of an interface between separated blood components within the separator 22. An exemplary optical system is shown in U.S. Patent Application Publication No. 2019/0201916, which is hereby incorporated herein by reference. An optical sensor 34 is also provided to optically monitor one or more conduits leading into or out of the separator 22.

The face of the processing device 10 includes a nesting module 36 for seating a flow control cassette 50 (FIG. 2) of the fluid flow circuit 12 (described in greater detail below). The cassette nesting module 36 is configured to receive various disposable cassette designs so that the system may be used to perform different types of procedures. Embedded within the illustrated cassette nesting module 36 are four valves 38a-d (collectively referred to herein as being part of the "valve system" of the processing device 10) for opening and closing fluid flow paths within the flow control cassette 50, and three pressure sensors 40a-c capable of measuring the pressure at various locations of the fluid flow circuit 12.

With reference to FIG. 2, the illustrated fluid flow circuit 12 includes a plurality of containers 42, 44, 46, and 48, with a flow control cassette 50 and a processing/separation chamber 52 that is configured to be received in the separator 22, all of which are interconnected by conduits or tubing segments, so as to permit continuous flow separation. The number of containers and associated tubing segments may vary depending on the procedure. The flow control cassette 50 routes the fluid flow through three tubing loops 54, 56, 58, with each loop being positioned to engage a particular one of the pumps 16, 18, 20. The conduits or tubing may extend through the cassette 50, or the cassette 50 may have pre-formed fluid flow paths that direct the fluid flow.

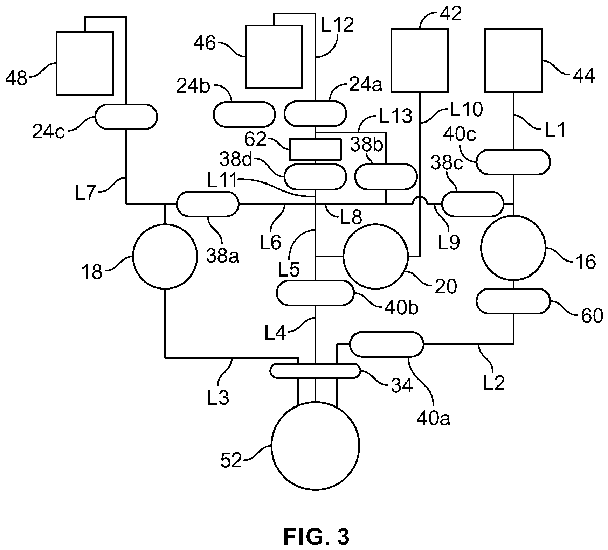

In the fluid flow circuit 12 shown in FIG. 2, container 42 may be pre-filled with additive solution, container 44 may be filled with whole blood and connected to the fluid flow circuit 12 at the time of use, container 46 may be an empty container for the receipt of red blood cells separated from the whole blood, and container 48 may be an empty container for the receipt of plasma separated from the whole blood. While FIG. 2 shows a whole blood container 44 (configured as a blood pack unit, for example) as a blood source, it is within the scope of the present disclosure for the blood source to be a living donor, as will be described in greater detail herein. The fluid flow circuit may optionally include an air trap 60 (FIG. 3) through which the whole blood is flowed prior to entering the separation chamber and/or a leukoreduction filter 62 through which the red blood cells are flowed prior to entering the red blood cell collection container 46.

The processing chamber 52 may be pre-formed in a desired shape and configuration by injection molding from a rigid plastic material, as shown and described in U.S. Patent No. 6,849,039, which is hereby incorporated herein by reference. The specific geometry of the processing chamber 52 may vary depending on the elements to be separated, and the present disclosure is not limited to the use of any specific chamber design. For example, it is within the scope of the present disclosure for the processing chamber 52 to be formed of a generally flexible material, rather than a generally rigid material. When the processing chamber 52 is formed of a generally flexible material, it relies upon the centrifuge 22 to define a shape of the processing chamber 52. An exemplary processing chamber formed of a flexible material and an associated centrifuge are described in U.S. Patent No. 6,899,666, which is hereby incorporated herein by reference.

In keeping with the disclosure, the controller of the processing device 10 is pre-programmed to automatically operate the system to perform one or more standard blood processing procedures selected by an operator by input to the touchscreen 14, and configured to be further programmed by the operator to perform additional blood processing procedures. The controller may be pre-programmed to substantially automate a wide variety of procedures, including, but not limited to: red blood cell and plasma production from a single unit of whole blood (as will be described in greater detail herein); red blood cell, plasma, and buffy coat product collection (as described in WO 2021/194824, previously incorporated herein); buffy coat pooling; buffy coat separation into a platelet product (as described in U.S. Patent Application Publication No. 2018/0078582, which is hereby incorporated herein by reference); glycerol addition to red blood cells; red blood cell washing (as described in WO 2021/194824, previously incorporated herein); platelet washing; and cryoprecipitate pooling and separation.

The pre-programmed blood processing procedures operate the system at preset settings for flow rates and centrifugation forces, and the programmable controller may be further configured to receive input from the operator as to one or more of flow rates and centrifugation forces for the standard blood processing procedure to override the pre-programmed settings.

In accordance with the present disclosure, the controller is configured to determine and to add a custom amount of additive solution to obtain a blood component product with a target collection parameter. The target collection parameter may be a pre-determined value and may be entered into the controller by the user. For example, in the collection of RBCs, it may be advantageous to obtain a red blood cell product with a target red blood cell product hematocrit. The target red blood cell hematocrit may be entered into the controller and the controller may operate the system to obtain an RBC product with the target RBC hematocrit. A more detailed description of obtaining a RBC product with a target RBC hematocrit is set forth below, yet it will be understood by one of ordinary skill in the art that the system may be employed to obtain other blood component products with target parameters by adding a custom amount of additive solution, for example, a platelet product with a target platelet concentration or platelet count.

In an embodiment, the controller may be configured to obtain a RBC product with a target RBC product hematocrit by continuously adding an additive solution to separated red blood cells. During the collection, the controller may determine a custom, determined additive flow rate $Q_{additive}$ and direct the additive pump 20 to introduce additive solution to the collected red blood cells at the determined flow rate.

The controller may determine $Q_{additive}$ based on values obtained by the controller from the various sensors and scales and other inputs associated with the system and the following equations:

$$Q_{RBC} = Q_{WB} - Q_P \qquad \text{[Equation 1]}$$

$$Q_{pure-RBC} = Q_{RBC} * H_{RBC} \qquad \text{[Equation 2]}$$

$$Q_{additive-RBC} = Q_{pure-RBC}/H_{product-RBC} \qquad \text{[Equation 3]}$$

$$Q_{additive} = Q_{additive-RBC} - Q_{RBC} \qquad \text{[Equation 4]}.$$

As shown in Equation 4, above, $Q_{additive}$ may be determined by the difference of the flow rate of the red blood cells mixed with additive solution ($Q_{additive-RBC}$) and the flow rate of packed red blood cells exiting the separator ($Q_{RBC}$). Packed red blood cells are red blood cells with (minimal) residual plasma.

$Q_{RBC}$ is determined by Equation 1, taking the difference of the flow rate of whole blood ($Q_{WB}$) being pumped into the separator by blood source pump 16 and the flow rate of plasma ($Q_P$) being pumped out of the separator by plasma pump 18. $Q_{WB}$ and $Q_P$ may be known rates as determined by the target pump rate of their respective pumps 16 and 18, pump rotation feedback, weight scale changes, or a combination of these flow rate monitoring methods. For example, $Q_{WB}$ and $Q_P$ may be pre-determined flow rates or may be determined by the controller based on data from the weight scales and/or the optical system.

Next, the controller may determine the flow rate of pure red blood cells ($Q_{pure-RBC}$) exiting the separator. $Q_{pure-RBC}$ is determined based on $Q_{RBC}$ and the hematocrit of the packed red blood cells ($H_{RBC}$) as shown in Equation 2. For instance, $Q_{pure-RBC}$ is obtained by multiplying $Q_{RBC}$ and $H_{RBC}$. $H_{RBC}$ may be assumed to be a constant empirically derived hematocrit for all procedures, such as 80%. Alternatively, $H_{RBC}$ may be estimated by an optical method using the optical sensors, or by any other applicable method without departing from the scope of the disclosure.

The controller may determine $Q_{additive-RBC}$ by dividing $Q_{pure-RBC}$ by the pre-determined target red blood cell product hematocrit ($H_{product-RBC}$).

Once the values for $Q_{additive-rbc}$ and $Q_{RBC}$ have been determined, the controller may use Equation 4 to determine the custom additive flow rate to obtain the target red blood cell product hematocrit. The controller may then direct the additive pump 20 to continuously pump the additive solution at the determined rate.

Alternatively, instead of determining $Q_{additive}$, the controller may be configured to obtain a RBC product with a target hematocrit by determining the volume of additive solution to be added to the red blood cell product. In this instance, the controller may direct the additive pump 20 to pump additive solution into the red blood cell container 46 after the red blood cells have been collected. In this case, Equation 5 can be used to determine $V_{additive}$. Equation 5 is similar to Equation 4, except volumes (V) are used instead of using flow rates (Q) in the calculations. For example:

$$V_{additive} = (V_{pure-RBC})/H_{product-RBC}) - V_{RBC} \qquad \text{[Equation 5]}.$$

The volume of pure red blood cells ($V_{pure-RBC}$) may be determined by multiplying the volume of blood to be processed, for instance, the whole blood collection volume, with the hematocrit of the blood to be processed. The volume of packed red blood cells ($V_{RBC}$) may be determined by dividing $V_{pure-RBC}$ by the packed red blood cell hematocrit ($H_{RBC}$).

Illustrative examples of the determination of $Q_{additive}$ and $V_{additive}$ are set forth below.

Example 1

In one example, 500 mL of whole blood with a hematocrit of 40% was processed to collect a red blood cell product with a target hematocrit of 58%. The whole blood was pumped into the centrifuge at a flow rate of 40 mL/min and plasma was pumped out of the centrifuge at a plasma flow rate of 20 mL/min. In this example, the $H_{RBC}$ was assumed to be 80%. $Q_{additive}$ was determined as follows:

$$Q_{RBC} = Q_{WB} - Q_P = 40 \text{ mL/min} - 20 \text{ mL/min} = 20 \text{ mL/min} \qquad \text{[Equation 1]}$$

$$Q_{pure-RBC} = Q_{RBC} * H_{RBC} = 20 \text{ mL/min} * 0.80 = 16 \text{ mL/min} \qquad \text{[Equation 2]}$$

$$Q_{additive-RBC} = Q_{pure-RBC}/H_{product-RBC} = (16 \text{ mL/min})/0.58 = 27.6 \text{ mL/min} \qquad \text{[Equation 3]}$$

$$Q_{additive} = Q_{additive-rbc} - Q_{RBC} = 27.6 \text{ mL/min} - 20 \text{ mL/min} = 7.6 \text{ mL/min} \qquad \text{[Equation 4]}.$$

Using the same values as presented in Example 1, $V_{pure-RBc}$ was 200 mL (500mL*0.40) and $V_{RBC}$ was 250 mL (200 mL/0.80). Accordingly, $V_{additive}$ was determined as follows:

$$V_{additive}=(V_{pure-RBC})/H_{product-RBC}) - V_{RBC} = (200 \text{ mL}/0.58) - 250 \text{ mL} = 94.8 \text{ mL} \text{ [Equation 5]}.$$

Example 2

In a second example, 500 mL of whole blood with a hematocrit of 50% was processed to collect a red blood cell product with a target hematocrit of 58%. The whole blood was pumped into the centrifuge at a flow rate of 40 mL/min and plasma was pumped out of the centrifuge at a plasma flow rate of 15 mL/min. In this example, the $H_{RBC}$ was assumed to be 80%. $Q_{additive}$ was determined as follows:

$$Q_{RBC} = Q_{WB} - Q_P = 40 \text{ mL/min} - 15 \text{ mL/min} = 25 \text{ mL/min} \qquad \text{[Equation 1]}$$

$$Q_{pure-RBC} = Q_{RBC} * H_{RBC} = 25 \text{ mL/min} * 0.80 = 20 \text{ mL/min} \qquad \text{[Equation 2]}$$

$$Q_{additive-RBC} = Q_{pure-RBC}/H_{product-RBC} = (20 \text{ mL/min})/0.58 = 34.5 \text{ mL/min} \qquad \text{[Equation 3]}$$

$$Q_{additive} = Q_{additive-rbc} - Q_{RBC} = 34.5 \text{ mL/min} - 25 \text{ mL/min} = 9.5 \text{ mL/min} \qquad \text{[Equation 4]}.$$

Using the same values as presented in Example 2, $V_{pure-RBc}$ was 250 mL (500mL*0.50) and $V_{RBC}$ was 312.5 mL (250 mL/0.80). Accordingly, $V_{additive}$ was determined as follows:

$$V_{additive} = (V_{pure-RBC})/H_{product-RBC}) - V_{RBC} = (250 \text{ mL}/0.58) - 312.5 \text{ mL} = 118.5 \text{ mL}$$

$$\text{[Equation 5]}.$$

Table 1 summarizes the obtained values when determining the volume of additive solution in the two provided examples and compares them to similar examples wherein a standard amount of additive solution was added to the red blood cell product.

Table 1

|  | Standard Volume of Additive (110 mL) | | Determined Volume of Additive | |
|---|---|---|---|---|
| Donation Volume and Hct. | 500 mL at 40% | 500 mL at 50% | 500 mL at 40% | 500 mL at 50% |
| Pure RBCs Volume | 200 mL | 250 mL | 200 mL | 250 mL |
| Packed RBCs @ 80% Hct. | 250 mL | 312.5 mL | 250 mL | 312.5 mL |
| Additive Solution Volume | 110 mL | 110 mL | 94.8 mL | 118.5 mL |
| Total RBC Product Volume | 360 mL | 422.5 mL | 360 mL | 422.5 mL |
| RBC Product Hct. | 55.6% | 59.2% | 58% | 58% |

As shown in Table 1, by providing a custom, determined volume of additive solution, a target red blood cell hematocrit can be obtained.

The processing device 10 including a controller configured to determine and add a custom amount of additive solution may be used with other collection and/or post collection procedures. The examples described below are merely illustrative and other collection and post collection procedures may be executed with the processing device 10 without departing from the scope of the disclosure. It will be understood that the methods and systems of adding a custom amount of additive solution to a collected RBC product will apply to the following RBC collection protocols wherein (1) RBC and plasma are collected and (2) RBC, plasma and buffy coat are collected. The custom addition of additive solution to the RBCs collected in such protocols will proceed in substantially the same way as previously described, with variations noted below,

Red Blood Cell And Plasma Product Collection

In an exemplary procedure, the processing device 10 and the fluid flow circuit 12 may be used in combination to process a unit of whole blood into a red blood cell product and a plasma product. The controller may be configured to separate red blood cells from a blood source and to collect a red blood cell product with a pre-determined, target hematocrit. FIG. 3 is a schematic illustration of the fluid flow circuit 12 mounted to the processing device 10, with selected components of the fluid flow circuit 12 and selected components of the processing device 10 being shown. As shown in FIG. 3, one of the clamps 24b is not used in producing the red blood cell and plasma products (but could be used in other procedures), while the other illustrated components of the processing device 10 are employed.

In an initial stage, which is referred to herein as a "blood prime" stage, selected components of the fluid flow circuit 12 are primed using blood from a blood source. This is in contrast to typical apheresis devices, which employ a separately provided fluid (e.g., anticoagulant or saline) to prime a fluid flow circuit. The blood source is shown in FIG. 3 as the whole blood container 44, but may alternatively be a living donor. Thus, it should be understood that the term "whole blood" may refer to blood that either includes or omits an anticoagulant fluid.

During the blood prime stage, whole blood is drawn into the fluid flow circuit 12 from the blood source (the whole blood container 44 in the embodiment of FIG. 3) via line L1 by operation of the first pump 16 (which may be referred to as the "whole blood pump"). Valve 38c is closed, which directs the blood through pressure sensor 40c and into line L2. The blood passes through air trap 60, pressure sensor 40a (which measures the pressure of the processing chamber 52), and optical sensor 34 before flowing into the processing chamber 52, which is positioned within the centrifuge 22 of the processing device 10.

The centrifuge 22 may be stationary during the blood prime stage or may instead be controlled by the controller of the processing device 10 to spin at a low rotation rate (e.g., on the order of approximately 1,000-2,000 rpm). It may be advantageous for the centrifuge 22 to rotate during the blood prime stage in order to create enough g-force to ensure that the air in the processing chamber 52 (which includes air already present in the processing chamber 52, along with air moved into the processing chamber 52 from lines L1 and/or L2 by the flow of blood) is forced towards the low-g (radially inner) wall of the processing chamber 52. Higher centrifuge rotation rates, such as 4,500 rpm (which is required for steady state separation, as will be described) may be undesirable as air blocks (in which air gets stuck and cannot be forced out of the processing chamber 52, causing pressure to rise) are more likely at higher g-forces.

The blood entering the processing chamber 52 will move towards the high-g (radially outer) wall of the processing chamber 52, displacing air towards the low-g wall. A plasma outlet port of the processing chamber 52 is associated with the low-g wall of the processing chamber 52, such that most of the air will exit the processing chamber 52 via the plasma outlet port and associated line L3, although some air may also exit the processing chamber 52 via a red blood cell outlet port associated with the high-g wall of the processing chamber 52.

Valves 38b and 38d are closed, while the second pump 18 (which may be referred to as the "plasma pump") is active and the third pump 20 (which may be referred to as the "additive pump") is inactive. Such an arrangement will direct the air exiting the processing chamber 52 via the red blood cell outlet port through associated line L4 and pressure sensor 40b, into line L5 and then into line L6. Valve 38a is open, such that the air flowing through line L6 will meet up with the air flowing through line L3 (i.e., the air that exits the processing chamber 52 via the plasma outlet port). The combined air will flow through line L7 and open clamp 24c, into the plasma collection container 48.

The flow of air out of the processing chamber 52 via either outlet port is monitored by the optical sensor 34, which is capable of determining the optical density of the fluid flowing through the monitored lines and discerning between air and a non-air fluid in lines L3 and L4. When a non-air fluid is detected in both lines L3 and L4, the controller of the processing device 10 will end the blood prime stage and move on to the next stage of the procedure. The amount of blood drawn into the fluid flow circuit 12 from the blood source during the blood prime stage will vary depending on a number of factors (e.g., the amount of air in the fluid flow circuit 12), but may be on the order of approximately 50 to 100 mL. The blood prime stage may take on the order of one to two minutes.

The next stage is referred to herein as the "establish separation" stage. Once non-air fluid has been detected in lines L3 and L4, the rotational speed of the centrifuge 22 will be increased to a rate that is sufficient to separate blood into packed red blood cells and platelet-poor plasma (which may be in the range of approximately 4,500 to 5,500 rpm, for example). To produce a plasma product that is low in platelets, it may be advantageous for the processing chamber 52 to be configured with a plasma outlet port that is spaced from and positioned downstream of the blood inlet port, rather than being positioned adjacent to the blood inlet port. Such a configuration allows the platelets to settle down into a distinct layer between the plasma and the red blood cells (commonly referred to as a "buffy coat") before the plasma is removed from the processing chamber 52, thus allowing the separated plasma to be platelet-depleted. As for the whole blood pump 16, it continues to operate, but no additional blood is drawn into the fluid flow circuit 12 from the blood source during the establish separation stage (as will be described).

As the blood source includes (in the case of a whole blood container) or provides (in the case of a living donor) only a single unit of whole blood (approximately 500 mL), the system must work with a finite fluid volume. To avoid product loss or quality issues, the plasma and red blood cells initially separated from the blood in the processing chamber 52 and removed from the processing chamber 52 are not directed to their respective collection containers, but are instead mixed together to form recombined whole blood and recirculated back into the processing chamber 52.

More particularly, during the establish separation stage, separated plasma will exit the processing chamber 52 via the plasma outlet port and associated line L3. Clamp 24c is closed during this stage, while valve 38a remains open, which directs the plasma from line L3 into line L6. Separated red blood cells exit the processing chamber 52 via the red blood cell outlet port and associated line L4. In the illustrated embodiment, there is no pump associated with line L4, such that the red blood cells exit the processing chamber 52 at a rate that is equal to the difference between the rate of the whole blood pump 16 and the rate of the plasma pump 18. In alternative embodiments, there may be a pump associated with the red blood cell outlet line instead of the plasma outlet line or a first pump associated with the plasma outlet line and a second pump associated with the red blood cell outlet line.

The additive pump 20 is inactive during this stage, thereby directing the red blood cells from line L4 into line L5. The plasma flowing through line L6 is mixed with the red blood cells flowing through line L5 at a junction of the two lines L5 and L6 to form recombined whole blood. Valve 38d is closed, which directs the recombined whole blood into line L8. Valve 38b is also closed, which directs the recombined whole blood from line L8 into line L9 and through open valve 38c. The whole blood pump 16 draws the recombined whole blood into line L2 from line L9 (rather than drawing additional blood into the fluid flow circuit 12 from the blood source), with the recombined blood passing through air trap 60, pressure sensor 40a, and optical sensor 34 before flowing back into the processing chamber 52, where it is again separated into plasma and red blood cells.

The establish separation stage continues until steady state separation has been achieved, which may take on the order of approximately one to two minutes. As used herein, the phrase "steady state separation" refers to a state in which blood is separated into its constituents in the processing chamber 52, with the radial position of the interface between separated components within the processing chamber 52 being at least substantially maintained (rather than moving radially inwardly or outwardly). The position of the interface may be determined and controlled according to any suitable approach, including using an interface detector of the type described in U.S. Patent Application Publication No. 2019/0201916.

Preferably, steady state separation is achieved with the interface between separated components within the processing chamber 52 at a target location. The target location may correspond to the location of the interface at which separation efficiency is optimized, with the precise location varying depending on a number of factors (e.g., the hematocrit of the whole blood). However, in an exemplary embodiment, the target location of the interface may be the position of the interface when approximately 52% of the thickness or width (in a radial direction) of the channel defined by the processing chamber 52 is occupied by red blood cells. In the illustrated embodiment, the position of the interface within the processing chamber 52 may be adjusted by changing the flow rate of the plasma pump 18, with the flow rate being increased to draw more separated plasma out of the processing chamber 52 (which decreases the thickness of the plasma layer within the processing chamber 52) and move the interface toward the low-g wall or decreased to draw less plasma out of the processing chamber 52 (which increases the thickness of the plasma layer within the processing chamber 52) and move the interface toward the high-g wall.

In an exemplary procedure, the controller of the processing device 10 will control the whole blood pump 16 to operate at a constant rate, with the plasma pump 18 initially operating at the same rate, which will quickly increase the thickness of the red blood cell layer within the processing chamber 52 and move the interface toward the low-g wall. The rate of the plasma pump 18 is gradually decreased as the thickness of the red blood cell layer increases and the location of the interface approaches the target location. As described above, the target location of the interface may depend upon the hematocrit of the whole blood, meaning that the rate of the plasma pump 18 (which controls the position of the interface) may also depend on the hematocrit of the whole blood. In one embodiment, this relationship may be expressed as follows:

Theoretical plasma pump rate = whole blood pump rate - ((whole blood hematocrit * whole blood pump rate) / hematocrit of separated red blood cells) [Equation 6]

The hematocrit of the whole blood may be measured before the procedure begins or by the optical sensor 34 during the procedure, while the hematocrit of the separated red blood cells may be determined during the procedure by the optical sensor 34 monitoring line L4. In practice, the plasma pump rate will typically not remain at the theoretical rate once steady state separation has been achieved, with the interface at the target location, but rather the plasma pump rate will instead tend to "flutter" around the theoretical rate.

Regardless of the particular manner in which the controller of the processing device 10 executes the establish separation stage and arrives at steady state separation, once steady state separation has been established, the controller ends the establish separation stage and advances the procedure to a "collection" stage. At the beginning of the collection stage, the centrifuge 22, the whole blood pump 16, and the plasma pump 18 all continue operating at the same rates at which they were operating at the end of the establish separation stage. The valve system of the processing device 10, however, is adjusted to direct the separated plasma and red blood cells to their respective collection containers (rather than recombining them and recirculating them through the centrifuge 22), while causing additional blood to be drawn into the fluid flow circuit 12 from the blood source until a total of one unit of whole blood has been drawn into the fluid flow circuit 12.

More particularly, during the collection stage, valve 38c is closed, which causes the whole blood pump 16 to draw additional blood into line L1 from the blood source (which is the whole blood container 44 in the illustrated embodiment, but may be a living donor). The whole blood pump 16 draws the blood from the blood source into line L2 from line L1, with the blood passing through air trap 60, pressure sensor 40a, and optical sensor 34 before flowing into the processing chamber 52, where it is separated into plasma and red blood cells. Most of the platelets of the whole blood will remain in the processing chamber 52, along with some white blood cell populations (much as mononuclear cells), while larger white blood cells, such as granulocytes, may exit with the packed red blood cells.

The separated plasma exits the processing chamber 52 via the plasma outlet port and associated line L3. Valve 38a is closed, which directs the plasma from line L3 into line L7, through open clamp 24c, and into the plasma collection container 48.

As for the separated red blood cells, they exit the processing chamber 52 via the red blood cell outlet port and associated line L4. The additive pump 20 is operated by the controller to draw an additive solution (such as, for example, ADSOL® in one exemplary embodiment, but may be some other red blood cell additive) from the additive solution container 42 via line L10. As described above, the controller may be configured to continuously add the additive solution to the separated red blood cells at a custom flow rate, $Q_{additive}$, to obtain a red blood cell product with a target hematocrit. The red blood cells flowing through line L4 are mixed with the additive solution flowing through line L10 at a junction of the two lines L4 and L10 to form a mixture that continues flowing into and through line L5.

The mixture is ultimately directed into the red blood cell collection container 46, but may first be conveyed through a leukoreduction filter 62 (if provided). Even if a leukoreduction filter 62 is provided, the valve system may be controlled to cause the mixture to bypass the leukoreduction filter 62 and enter the red blood cell collection container 46 without being leukoreduced. It is also within the scope of the present disclosure for the mixture to be routed through the leukoreduction filter 62 at the beginning of the collection stage, with the valve system being reconfigured during the collection stage to cause the mixture to bypass the leukoreduction filter 62, such that only a portion of the collected red blood cells are leukoreduced. A more detailed description of the configuration of the system during leukoreduction is provided in WO 2021/194824, which has been previously incorporated herein.

Regardless of whether the collected red blood cells have been leukoreduced (or only partially leukoreduced), the collection stage continues until one unit of whole blood has been drawn into the fluid flow circuit 12 from the blood source. In the case of a whole blood container 44 being used as a blood source (as in the illustrated embodiment) the collection stage will end when the whole blood container 44 (which is initially provided with one unit of whole blood) is empty, with different approaches possibly being employed to determine when the whole blood container 44 is empty. For example, in one embodiment, pressure sensor 40c monitors the hydrostatic pressure of the whole blood container 44. An empty whole blood container 44 may be detected when the hydrostatic pressure measured by pressure sensor 40c is at or below a threshold value. Alternatively (or additionally), the weight of the whole blood container 44 may be monitored by a weight scale, with an empty whole blood container 44 being detected when the weight is at or below a threshold value. In the case of a living donor (or in the event that the whole blood container 44 is provided with more than one unit of blood), the volumetric flow rate of the whole blood pump 16 may be used to determine when one unit of whole blood has been drawn into the fluid flow circuit 12.

Once a total of one unit of whole blood has been drawn into the fluid flow circuit 12, the controller will transition the procedure to a "red blood cell recovery" stage. During the red blood cell recovery stage, air from the plasma collection container 48 (which was conveyed there during the blood prime stage) is used to recover the contents of the processing chamber 52 (which may be primarily red blood cells) to reduce product loss.

In an embodiment, the whole blood pump 16 is deactivated, while the plasma pump 18 is operated in a reverse direction (with respect to its direction of operation up to this stage of the procedure). This draws the air from the plasma collection container 48 and into line L7. Valve 38a is closed, while clamp 24c is open, which directs the air through line L7, into and through line L3, and into the processing chamber 52 via the plasma outlet port. On account of the air flowing through the plasma outlet port, it will enter the processing chamber 52 at the low-g side. As additional air is introduced into the processing chamber 52, it will move from the low-g wall towards the high-g wall, thus displacing any liquid content through the red blood cell outlet port at the high-g side and into line L4. During this stage, the centrifuge 22 may be operated at a slower rate (e.g., in the range of approximately 1,000-2,000 rpm) to decrease the risk of an air blockage (as during the blood prime stage).

The additive pump 20 continues its operation, drawing additive solution from the additive solution container 42 and through line L10, to be mixed with the contents of the processing chamber 52 flowing through line L4 at the junction of the two lines L4 and L10. In an embodiment, the RBCs exiting the processing chamber 52 during the recovery stage contain approximately the same hematocrit as the RBCs exiting the processing chamber 52 during the collection stage, and accordingly, the additive pump rate during the recovery stage may be the same as in the collection stage. The mixture continues flowing into and through line L5. If the valve system was arranged so as to direct flow through the leukoreduction filter 62, valves 38a, 38b, and 38c may remain closed, with valve 38d being open to direct the mixture into line L11 for leukoreduction. On the other hand, if the valve system was arranged so as to bypass the leukoreduction filter 62, valves 38a, 38c, and 38d may remain closed, with valve 38b being open to direct the mixture through lines L8 and L13 to bypass the leukoreduction filter 62. As described above with regard to the collection stage, it is possible for the controller to change the configurations of the valve system during the red blood cell recovery stage to stop leukoreduction of the mixture (e.g., if the pressure of the leukoreduction filter 62 becomes too great).

Regardless of whether the mixture is filtered, it flows into line L12, through open clamp 24a, and into the red blood cell collection container 46. The red blood cell recovery stage continues until all of the air is removed from the plasma collection container 48. In one exemplary embodiment, the weight of the plasma collection container 48 may be monitored by a weight scale, with an empty plasma collection container 48 being detected when the weight is at or below a threshold value. Other approaches may also be employed to determine when to end the red blood cell recovery stage, such as using the optical sensor 34 to detect plasma flowing through line L3.

Once the red blood cell recovery stage is complete, the procedure may transition to an "additive solution flush" stage. During the additive solution flush stage, additive solution from the additive solution container 42 is conveyed into the red blood cell collection container 46 until a determined amount of additive solution is in the red blood cell collection container 46.

As described herein, the controller is configured to determine and to add a custom volume of additive solution to obtain a red blood cell product with a target hematocrit. In an embodiment, additive solution is not added to the red blood cells during the collection stage, and is instead added during the additive flush stage. For instance, the controller may direct the system to introduce a determined volume of additive solution ($V_{additive}$) to obtain a target RBC product hematocrit. Alternatively, the additive solution may be added during both the collection stage and the additive flush stage. In this instance, the controller may supplement the amount of additive solution introduced to the red blood cells during the collection stage with an additional custom amount of additive solution during the additive solution flush stage to obtain a RBC product with a target hematocrit. The only change in transitioning from the red blood cell recovery stage to the additive solution flush stage involves deactivating the plasma pump to prevent plasma from being removed from the plasma collection container 48 (though it is also possible for the additive pump 20 to operate at a different rate).

The additive solution flush stage will continue until the determined amount of additive solution has been added to the red blood cell collection container 46. In one exemplary embodiment, the weight of the additive solution container 42 may be monitored by a weight scale, with a particular change in weight corresponding to the determined amount of additive solution having been conveyed to the red blood cell collection container 46. Alternatively (or additionally), the weight of the red blood cell collection container 46 may be monitored by a weight scale, with a particular change in weight corresponding to the determined amount of additive solution having been conveyed to the red blood cell collection container 46.

After completion of the red blood cell recovery stage or the additive solution flush stage, the system may transition to an "air evacuation" stage. During the air evacuation stage, the red blood cell collection container 46 is "burped" to remove all residual air for storage (just as air was removed from the plasma collection container 48 during the red blood cell recovery stage). This is done by reversing the direction of operation of the additive pump 20, closing valve 38d (if not already closed at the end of the additive solution flush stage), and opening valve 38b (if not already open at the end of the additive solution flush stage). The additive pump 20 draws air out of the red blood cell collection container 46, through line L12 and open clamp 24a, into line L13 and through open valve 38b. The air continues through line L8, line L5, and line L10, with the air ending up in the additive solution container 42. In an embodiment, air may be evacuated from the red blood cell collection container 46 to the additive solution container 42. It is also within the scope of the present disclosure for all or a portion of the air to be directed to a different location of the fluid flow circuit 12 (e.g., into the processing chamber 52 and/or into the whole blood container 44, if provided).

The air evacuation stage will continue until all of the air is removed from the red blood cell collection container 46, which may be determined (for example) by detecting a change in the weight of the red blood cell collection container 46 (e.g., using a weight scale).

Upon completion of the air evacuation stage, any of a number of post-processing stages may be executed. For example, the system may execute a "sealing" stage in which all of the clamps and valves are closed and all of the pumps are deactivated. The line L12 connected to the red blood cell collection container 46 and the line L7 connected to the plasma collection container 48 are sealed and optionally severed for storage of the plasma and red blood cell products. If lines L7 and L12 are severed, the plasma collection container 48 and the red blood cell collection container 46 may be stored, while the remainder of the fluid flow circuit 12 is disposed of. Lines L7 and L12 may be sealed (and optionally severed) according to any suitable approach, which may include being sealed by RF sealers incorporated or associated with clamps 24a and 24c, for example. In another embodiment, the fluid flow circuit 12 may be removed from the processing device 10, with lines L7 and L12 being sealed (and optionally severed) using a dedicated sealing device.

Red Blood Cell, Plasma, And Buffy Coat Product Collection

In another exemplary procedure, the processing device 10, as described herein, may be used in a RBC, plasma, and buffy coat product collection. For example, the processing device 10 may be used in combination with a suitably configured fluid flow circuit to process a unit of whole blood into separate red blood cell, plasma, and buffy coat products.

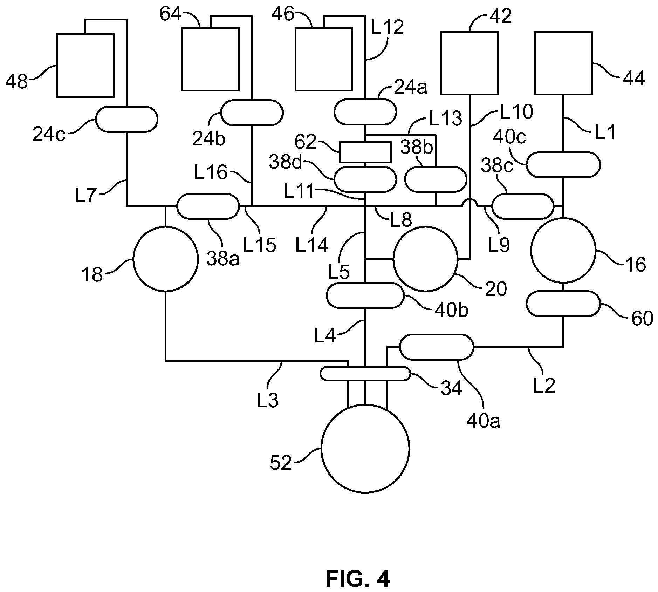

An exemplary fluid flow circuit is shown in FIG. 4, which also shows selected components of the processing device 10. Due to the similarity between the fluid flow circuits of FIGS. 3 and 4, the components of the fluid flow circuit of FIG. 4 corresponding to above-described components of the fluid flow circuit of FIG. 3 are identified using the same reference numbers, while new or differently configured components are identified using new reference numbers. In short, the principal difference between the fluid flow circuit of FIG. 3 and the fluid flow circuit of FIG. 4 is that line L6 of the fluid flow circuit of FIG. 3 is replaced in the fluid flow circuit of FIG. 4 with a pair of lines L14 and L15 that meet at a junction, with a third line L16 leading from the junction to a buffy coat collection container 64. Whereas clamp 24b of the processing device 10 is not used with the fluid flow circuit of FIG. 3, it will be seen that it is associated with line L16 of the fluid flow circuit of FIG. 4 and used during an exemplary procedure that will be described in greater detail below.

Many of the stages in a RBC, plasma, and buffy coat product collection procedure are similar to above-described stages of the RBC and plasma collection procedure.

The procedure begins with a "blood prime" stage that corresponds to the blood prime stage described above, with selected components of the fluid flow circuit being primed using blood from a blood source. In accordance with the above description of the procedure the blood source is shown in FIG. 4 as the whole blood container 44, but may alternatively be a living donor.

During the blood prime stage, whole blood is drawn into the fluid flow circuit from the blood source via line L1 by operation of the whole blood pump 16. Valve 38c is closed, which directs the blood through pressure sensor 40c and into line L2. The blood passes through air trap 60, pressure sensor 40a, and optical sensor 34 before flowing into the processing chamber 52, which is positioned within the centrifuge 22 of the processing device 10. In accordance with the above description of the blood prime stage of FIG. 3, the centrifuge 22 may be stationary during the blood prime stage or may be spun at a low rate (e.g., on the order of approximately 1,000-2,000 rpm).

The blood entering the processing chamber 52 will move towards the high-g (radially outer) wall of the processing chamber 52, displacing air towards the low-g wall. Most of the air will exit the processing chamber 52 via the plasma outlet port and associated line L3, although some air may also exit the processing chamber 52 via the red blood cell outlet port and associated line L4.

Valves 38b and 38d are closed, while the plasma pump 18 is active and the additive pump 20 is inactive. This directs the air exiting the processing chamber 52 via the red blood cell outlet port through associated line L4 and pressure sensor 40b, into line L5 and then into line L14. Clamp 24b is closed, while valve 38a is open, such that the air flowing through line L14 will flow into line L15 and then meet up with the air flowing through line L3 (i.e., the air that exits the processing chamber 52 via the plasma outlet port). The combined air will flow through line L7 and open clamp 24c, into the plasma collection container 48. The blood prime stage continues until a non-air fluid is detected in both lines L3 and L4.

The next stage is an "establish separation" stage that corresponds to the establish separation stage described above. In accordance with the above description of the establish separation stage, the rotational speed of the centrifuge 22 is increased to a rate that is sufficient to separate blood into packed red blood cells and platelet-poor plasma, with the buffy coat therebetween (e.g., in the range of approximately 4,500 to 5,500 rpm), while the whole blood pump 16 continues operating (but without drawing additional blood into the fluid flow circuit from the blood source).

Separated plasma will exit the processing chamber 52 via the plasma outlet port and associated line L3. Clamps 24b and 24c are closed during this stage, while valve 38a remains open, which directs the plasma from line L3 into line L15 and then into line L14. Separated red blood cells exit the processing chamber 52 via the red blood cell outlet port and associated line L4, while the buffy coat remains in the processing chamber 52. As explained above, there is no pump shown in association with line L4 (such that the red blood cells exit the processing chamber 52 at a rate that is equal to the difference between the rate of the whole blood pump 16 and the rate of the plasma pump 18), though it is within the scope of the present disclosure for there to be a pump associated with the red blood cell outlet line instead of the plasma outlet line or a first pump associated with the plasma outlet line and a second pump associated with the red blood cell outlet line.

The additive pump 20 is inactive during this stage, thereby directing the red blood cells from line L4 into line L5. The plasma flowing through line L14 is mixed with the red blood cells flowing through line L5 at the junction of the two lines L5 and L14 to form recombined whole blood. Valve 38d is closed, which directs the recombined whole blood into line L8. Valve 38b is also closed, which directs the recombined whole blood from line L8 into line L9 and through open valve 38c. The whole blood pump 16 draws the recombined whole blood into line L2 from line L9 (rather than drawing additional blood into the fluid flow circuit 12 from the blood source), with the recombined blood passing through air trap 60, pressure sensor 40a, and optical sensor 34 before flowing back into the processing chamber 52, where it is again separated into plasma and red blood cells, with the buffy coat therebetween.

The establish separation stage continues until steady state separation has been achieved, preferably with the interface between the separated plasma and the separated red blood cells and buffy coat within the processing chamber 52 at a target location. An exemplary approach for moving the interface to a target location within the processing chamber 52 is described above in greater detail with regard to the establishment of the separation stage.

Once steady state separation has been established, the controller ends the establish separation stage and advances the procedure to a "collection" stage that corresponds to the collection stage described above. At the beginning of the collection stage, the centrifuge 22, the whole blood pump 16, and the plasma pump 18 all continue operating at the same rates at which they were operating at the end of the establish separation stage. The valve system of the processing device 10, however, is adjusted to direct the separated plasma and red blood cells to their respective collection containers (rather than recombining them and recirculating them through the centrifuge 22), while causing additional blood to be drawn into the fluid flow circuit from the blood source until a total of one unit of whole blood has been drawn into the fluid flow circuit. As in the establish separation stage, the buffy coat remains in the processing chamber 52 during the collection stage.

More particularly, during the collection stage, valve 38c is closed, which causes the whole blood pump 16 to draw additional blood into line L1 from the blood source. The whole blood pump 16 draws the blood from the blood source into line L2 from line L1, with the blood passing through air trap 60, pressure sensor 40a, and optical sensor 34 before flowing into the processing chamber 52, where it is separated into plasma, red blood cells, and buffy coat.

The separated plasma exits the processing chamber 52 via the plasma outlet port and associated line L3. Valve 38a is closed, which directs the plasma from line L3 into line L7, through open clamp 24c, and into the plasma collection container 48.

The separated red blood cells exit the processing chamber 52 via the red blood cell outlet port and associated line L4. The additive pump 20 is operated by the controller to draw an additive solution (which may be ADSOL® or some other red blood cell additive) from the additive solution container 42 via line L10 in much the same manner as the Red Blood cell and Plasma Collection process described above, As also described above, the controller may be configured to continuously add the additive solution to the separated red blood cells at a custom flow rate, $Q_{additive}$, to obtain a red blood cell product with a target hematocrit. The red blood cells flowing through line L4 are mixed with the additive solution flowing through line L10 at a junction of the two lines L4 and L10 to form a mixture that continues flowing into and through line L5.

The mixture is ultimately directed into the red blood cell collection container 46, but may first be conveyed through a leukoreduction filter 62 (if provided). Even if a leukoreduction filter 62 is provided, the valve system may be controlled to cause the mixture to bypass the leukoreduction filter 62 and enter the red blood cell collection container 46 without being leukoreduced. It is also within the scope of the present disclosure for the mixture to be routed through the leukoreduction filter 62 at the beginning of the collection stage, with the valve system being reconfigured during the collection stage to cause the mixture to bypass the leukoreduction filter 62, such that only a portion of the collected red blood cells are leukoreduced.

The collection stage continues until one unit of whole blood has been drawn into the fluid flow circuit from the blood source. In the case of a whole blood container 44 being used as a blood source the collection stage will end when the whole blood container 44 (which is initially provided with one unit of whole blood) is empty. In the case of a living donor (or in the event that the whole blood container 44 is provided with more than one unit of blood), the volumetric flow rate of the whole blood pump 16 may be used to determine when one unit of whole blood has been drawn into the fluid flow circuit.

Once a total of one unit of whole blood has been drawn into the fluid flow circuit, the controller will transition the procedure to a "red blood cell recovery" stage. During the red blood cell recovery stage, air from the plasma collection container 48 (which was conveyed there during the blood prime stage) is used to recover separated red blood cells remaining in the processing chamber 52 without removing the buffy coat from the processing chamber 52.

In the illustrated embodiment, the whole blood pump 16 is deactivated, while the plasma pump 18 is operated in a reverse direction (with respect to its direction of operation up to this stage of the procedure). This draws the air from the plasma collection container 48 and into line L7. Valve 38a is closed, while clamp 24c is open, which directs the air through line L7, into and through line L3, and into the processing chamber 52 via the plasma outlet port. On account of the air flowing through the plasma outlet port, it will enter the processing chamber 52 at the low-g side. As additional air is introduced into the processing chamber 52, it will move from the low-g wall towards the high-g wall, thus displacing the separated red blood cells in the processing chamber 52 through the red blood cell outlet port at the high-g side and into line L4. During this stage, the centrifuge 22 may be operated at a slower rate (e.g., in the range of approximately 1,000-2,000 rpm) to decrease the risk of an air blockage.

The additive pump 20 continues its operation, drawing additive solution from the additive solution container 42 and through line L10, to be mixed with the red blood cells flowing through line L4 at the junction of the two lines L4 and L10. In an embodiment, the RBCs exiting the processing chamber 52 during the recovery stage contain approximately the same hematocrit as the RBCs exiting the processing chamber 52 during the collection stage, and accordingly, the additive pump rate during the recovery stage may be the same as in the collection stage. The mixture continues flowing into and through line L5. If the valve system was arranged in the so as to direct flow through the leukoreduction filter 62, valves 38a, 38b, and 38c may remain closed, with valve 38d being open to direct the mixture into line L11 for leukoreduction. On the other hand, if the valve system was arranged so as to bypass the leukoreduction filter 62, valves 38a, 38c, and 38d may remain closed, with valve 38b being open to direct the mixture through lines L8 and L13 to bypass the leukoreduction filter 62. As described above with regard to the collection stage, it is possible for the controller to change the configurations of the valve system during the red blood cell recovery stage to stop leukoreduction of the mixture (e.g., if the pressure of the leukoreduction filter 62 becomes too great).

Regardless of whether the mixture is filtered, it flows into line L12, through open clamp 24a, and into the red blood cell collection container 46. The red blood cell recovery stage continues until the red blood cells have been removed from the processing chamber 52. This may be determined in any of a number of ways without departing from the scope of the present disclosure. In one embodiment, the red blood cell recovery stage continues until a predetermined volume of fluid (corresponding to the volume of red blood cells remaining in the processing chamber 52) has been conveyed out of the processing chamber 52. This volume may be calculated for example, by determining the volume of red blood cells present in the one unit of whole blood (which may be determined based on the hematocrit of the blood) and then subtracting the volume of red blood cells that have already been conveyed into the red blood cell collection container 46 (which may be determined based on the weights of the red blood cell container and the additive solution container 42 at the end of the collection stage). In another embodiment, the red blood cell recovery stage may continue until the optical sensor 34 detects a non-red blood cell fluid (e.g., the buffy coat) flowing through line L4.

Some air will remain in the plasma collection container 48 at the end of the red blood cell recovery stage. At least a portion of the air remaining in the plasma collection container 48 is used in a "buffy coat harvest" stage to convey the buffy coat in the processing chamber 52 into the buffy coat collection container 64 as a buffy coat product. Due to air being conveyed through the processing chamber 52 during the buffy coat harvest stage, the centrifuge 22 may continue operating at a relatively low rate (e.g., in the range of approximately 1,000-2,000 rpm, as during the red blood cell recovery stage) to decrease the risk of an air blockage.

To transition from the red blood cell recovery stage to the buffy coat harvest stage, clamp 24b is opened, while clamp 24a is closed. One of valves 38b and 38d will be open at the end of the red blood cell recovery stage, while the other is closed (depending on whether the mixture of red blood cells and additive solution is routed through or around the leukoreduction filter 62. Whichever of the two valves 38b and 38d is open at the end of the red blood cell recovery stage is closed upon transitioning to the buffy coat harvest stage, such that both valves 38b and 38d are closed during the buffy coat harvest stage. The additive pump 20 is also stopped upon transitioning to the buffy coat harvest stage.

The plasma pump 18 will continue drawing air from the plasma collection container 48 into the processing chamber 52 via lines L7 and L3. As in the red blood cell recovery stage, the air will enter the processing chamber 52 at the low-g side and move from the low-g wall toward the high-g wall as additional air is conveyed into the processing chamber 52. This displaces the buffy coat remaining in the processing chamber 52 through the red blood cell outlet port at the high-g side and into line L4. The buffy coat is directed from line L4 into line L5 and then into line L14. From line L14, the buffy coat is directed into line L16, through open clamp 24b, and into the buffy coat collection container 64 as a buffy coat product.

The buffy coat harvest stage continues until the buffy coat has been removed from the processing chamber 52. This may be determined in any of a number of ways without departing from the scope of the present disclosure. In one embodiment, the buffy coat harvest stage continues until a predetermined volume of fluid (corresponding to the volume of the buffy coat or interface in the processing chamber 52) has been conveyed out of the processing chamber 52. This volume may be calculated for example, by subtracting the volumes of collected plasma and collected red blood cells at the end of the red blood cell recovery stage from the volume of blood that has been processed. In another embodiment, the buffy coat harvest stage may continue until the optical sensor 34 detects a non-buffy coat fluid (e.g., air) flowing through line L4.

Once the buffy coat harvest stage is complete, the procedure will transition to an "additive solution flush" stage. During the additive solution flush stage as described above, additive solution from the additive solution container 42 is conveyed into the red blood cell collection container 46 until a determined amount of additive solution is in the red blood cell collection container 46. As described herein, the controller is configured to determine and add a custom volume of additive solution to obtain a red blood cell product with a target hematocrit. In an embodiment, additive solution is not added to the red blood cells during the collection stage, and is instead added during the additive flush stage. For instance, the controller may direct the system to introduce a determined volume of additive solution ($V_{additive}$) to obtain a target RBC product hematocrit. Alternatively, the additive solution may be added during both the collection stage and the additive flush stage. In this instance, the controller may supplement the amount of additive solution introduced to the red blood cells during the collection stage with an additional custom amount of additive solution during the additive solution flush stage to obtain a RBC product with a target hematocrit.

To transition from the buffy coat harvest stage to the additive solution flush stage, clamp 24b is closed, clamp 24a is opened, the plasma pump 18 is deactivated, and the additive pump 20 is reactivated. Additionally, one of valves 38b and 38d is opened, which determines whether the additive solution will be conveyed through the leukoreduction filter 62 or routed around the leukoreduction filter 62 and which may depend on which of the two valves 38b and 38d was open at the end of the red blood cell recovery stage. Typically, if valve 38d was open at the end of the red blood cell recovery stage, it will be opened at the beginning of the additive solution flush stage to allow for red blood cells remaining in the leukoreduction filter 62 to be recovered by additive solution being conveyed through the leukoreduction filter 62. Similarly, if valve 38b was open at the end of the red blood cell recovery stage, it will be opened at the beginning of the additive solution flush stage to direct additive solution around the leukoreduction filter 62. However, it is also within the scope of the present disclosure for valve 38b to be closed and valve 38d to be open at the end of the red blood cell recovery stage, with valve 38b being open and valve 38d being closed at the beginning of the additive solution flush stage, if the controller determines that it is advisable to begin bypassing the leukoreduction filter 62. Additionally, it is within the scope of the present disclosure for the valve system to be arranged to direct additive solution through the leukoreduction filter 62 and to transition into a configuration to cause the additive solution to bypass the leukoreduction filter 62.

The additive solution flush stage will continue until a determined amount of additive solution has been added to the red blood cell collection container 46. When the additive solution flush stage is complete, the system will transition to an "air evacuation" stage. During the air evacuation stage, the red blood cell collection container 46 is "burped" to remove all residual air for storage (just as air was removed from the plasma collection container 48 during the red blood cell recovery and buffy coat harvest stages). This is done by reversing the direction of operation of the additive pump 20, closing valve 38d (if not already closed at the end of the additive solution flush stage), and opening valve 38b (if not already open at the end of the additive solution flush stage). The additive pump 20 draws air out of the red blood cell collection container 46 and into the additive solution container 42, in accordance with the above description of the air evacuation. While the air may be evacuated from the red blood cell collection container 46 to the additive solution container 42, it is within the scope of the present disclosure for all or a portion of the air to be directed to a different location of the fluid flow circuit.

The air evacuation stage will continue until all of the air is removed from the red blood cell collection container 46. Upon completion of the air evacuation stage, any of a number of post-processing stages may be executed. For example, a "sealing" stage in which all of the clamps and valves are closed and all of the pumps are deactivated may be executed. The line L12 connected to the red blood cell collection container 46, the line L7 connected to the plasma collection container 48, and the line L16 connected to the buffy coat collection container 64 are sealed and optionally severed for storage of the plasma, red blood cell, and buffy coat products. While it is possible to remove air from the buffy coat collection container 64 (e.g., using a variation of the air evacuation stage) before sealing line L16, doing so is typically not necessary, as it is most common for the collected buffy coat to be pooled with other buffy coats, with the pooled buffy coats being processed to create a platelet product.

Other Procedures

The methods and systems described above may be employed in executing other blood component collection/post-processing procedures. For example, a method and/or system utilizing a processing device including a controller configured to determine and add a custom amount of an additive solution, such as device 10 described herein, may be used in other blood processing procedures.

For example, a custom amount of additive solution may be added during a RBC washing procedure. Red blood cells are often stored for long periods of time prior to transfusion. In this case, the cells are commonly "washed" prior to transfusion to remove the supernatant suspension (and, thus, any undesirable extracellular substances) from the cells while maintaining the integrity and functionality of the cells, thereby decreasing the probability of immunological reaction in a recipient.

In certain situations, it may be desirable to change the hematocrit of a RBC product from pre- to post-wash to obtain a RBC product with a target hematocrit. For instance, during a RBC wash procedure, a controller may determine an amount of additive solution to add to the previously separated RBCs to obtain a RBC product with a target hematocrit. The additive solution may be added at a determined flow rate, $Q_{additive}$, or as a determined volume, $V_{additive}$, in the manner described above. For example, a prewash RBC product may contain a HCT of 58% and a total volume of 360 mL. It may be desirable for a post-wash RBC product, which will be transfused to a patient, to contain less overall volume and thus less additive solution. The target hematocrit of a post-wash RBC product may be 70% and a flow rate $Q_{additive}$ can be determined as explained above to achieve the desired hematocrit.

In an embodiment, a determined amount of additive solution may be added during a washing, RBC recovery, and/or dilution stage. A more detailed description of a RBC wash procedure is provided in WO 2021/194824, which has been previously incorporated herein.

In another example, the methods and systems described herein may be employed to add a custom amount of additive solution, for instance, a platelet additive solution (PAS), during a platelet collection procedure. The controller may be configured to collect platelets and to add a custom amount of PAS to obtain a platelet product with a target collection parameter. Examples of suitable platelet additive solutions are described in U.S. Patent No. 9,402,866, the contents of which is incorporated herein by reference.

In an embodiment, the system and platelet collection procedure may be the system a procedure described in U.S. Patent No. 11,465,169, filed on September 15, 2017, which is hereby incorporated in its entirety herein. During platelet collection, it may be desirable to collect a platelet product with a target concentration (often measured in units of platelets per microliter (plts/μL)) or a target platelet count. The platelet collection system may include a controller configured to determine and add a custom amount of additive solution, as described herein. The controller may be associated with the reusable hardware device. In an embodiment, the concentration of a fluid, such as platelet rich plasma (PRP) entering a separator, such as a spinning membrane, may be assumed to be a constant empirically derived value or it may be estimated by an optical method using the optical sensors, or by any other applicable method without departing from the scope of the disclosure. Based on the flow rates of the system, the controller may determine the platelet concentration exiting the separator, and then add a custom amount of PAS to achieve a desired final platelet product concentration or platelet count.

In an embodiment, the controller may be configured to obtain a desired platelet product concentration or platelet count by determining a custom PAS flow rate ($Q_{PAS}$). $Q_{PAS}$ can be determined using similar equations as Equations 2-4 described above, except, in place of hematocrit which is in terms of percentage, a platelet concentration ($C_{PLTs}$) is applied in term of cells/volume. For instance, the controller may determine $Q_{PAS}$ based on values obtained by the controller from various sensors, scales, and other inputs associated with the system and the following equations:

$$Q_{pure-PLTs} = Q_{PLTs} * C_{PLTs} \qquad \text{[Equation 6]}$$

$$Q_{PAS-PLTS} = \frac{Q_{pure-PLTs}}{Target\ PLT\ Concentration} \qquad \text{[Equation 7]}$$

$$Q_{PAS} = Q_{PAS-PLTs} - Q_{PLTs} \qquad \text{[Equation 8]}$$

As shown in Equation 8, above, $Q_{PAS}$ may be determined by the difference of the flow rate of the platelets mixed with PAS ($Q_{PAS-PLTs}$) and the flow rate of platelets exiting the separator ($Q_{PLTs}$).

When collecting a platelet product, $Q_{PLTs}$ may be the same as the flow rate of plasma ($Q_P$) being pumped out of the separator, as platelets will exit the separator with plasma. $Q_P$ may be a known rate as determined by the target pump rate of their respective pumps, pump rotation feedback, weight scale changes, or a combination of these flow rate monitoring methods. For example, $Q_P$ may be a pre-determined flow rate or may be determined by the controller based on data from the weight scales and/or the optical system. $Q_{PLTs}$ may also be a known value.

The controller may determine the flow rate of pure platelets ($Q_{pure-PLTs}$) exiting the separator. $Q_{pure-PLTs}$ is determined based on $Q_{PLTs}$ and the platelet concentration ($C_{PLTs}$) as shown in Equation 6. $Q_{pure-PLTs}$ is a count of total cells flowing rather than the volume of cells flowing which is the case for $Q_{pure-RBC}$, above. $Q_{pure-PLTs}$ is obtained by multiplying

$Q_{PLTs}$ and $C_{PLTs}$. $C_{PLTs}$ may be assumed to be a constant empirically derived concentration for all procedures or $C_{PLTs}$ may be estimated by an optical method using the optical sensors, or by any other applicable method without departing from the scope of the disclosure.

The controller may determine $Q_{PAS-PLTs}$ by dividing $Q_{pure-PLTs}$ by the pre-determined target platelet concentration.

Once the values for $Q_{PAS-PLTs}$ and $Q_{PLTs}$ have been determined, the controller may use Equation 8 to determine the custom additive flow rate to obtain the target platelet concentration. The controller may then direct the additive pump to continuously pump the additive solution at the determined rate.

An illustrative example of the determination of $Q_{PLTs}$ is set forth below.

Example 3

In an example where $Q_{PLTs}$ is 5 mL/min and $C_{PLTs}$ is 4500e3 platelets/$\mu$L, to obtain a target platelet concentration of 1500e3 platelets/$\mu$L, $Q_{PAS}$ was determined to be 10 mL/min as follows:

$$Q_{pure-PLTs} = 5\frac{ml}{min} * 4500e3\frac{PLTs}{uL} = 2.25e10 \; PLTs/min \quad \text{[Equation} \qquad 6]$$

$$Q_{PAS-PLTS} = \frac{2.25e10 \; PLTs/min}{1500e3/uL} = 15 \; ml/min \qquad \text{[Equation} \qquad 7]$$

$$Q_{PAS} = 15\frac{ml}{min} - 5 \; ml/\min = 10 \; ml/min \qquad \text{[Equation 8].}$$

Alternatively, instead of determining $Q_{PAS}$, the controller may be configured to obtain a platelet product with a target platelet concentration by determining the volume of PAS to be added to the platelet product. In this instance, the controller may direct the additive pump to pump PAS into a product container after the platelets have been collected. In this case, Equation 9 can be used to determine $V_{PAS}$, wherein the quotient of the total amount of collected platelets divided by the target platelet concentration is subtracted by the volume of platelets. For example:

$$V_{PAS} = \text{(Total PLT Count/Target PLT Concentration)} - V_{PLTs} \qquad \text{[Equation 9].}$$

There are additional aspects to the devices and methods described herein including, without limitation the following aspects.

Aspect 1. A system for collecting a blood component including a durable hardware component including a separator and a controller, wherein the controller is configured to automatically operate the system to separate a target blood component from a blood source, and determine and add a custom amount of additive solution to the target blood component to obtain a blood component product with a target collection parameter; and a disposable fluid flow circuit for mounting onto said durable hardware component including a separation chamber, and a plurality of containers in fluid communication with the separation chamber, wherein the plurality of containers includes at least a product container configured to contain the blood component product and an additive solution container configured to contain an additive solution.

Aspect 2. The system of Aspect 1, wherein the blood source includes whole blood or a previously separated blood component.

Aspect 3. The system of Aspect 2, wherein the durable hardware component includes at least one weight scale associated with the plurality of containers.

Aspect 4. The system of Aspect 3, wherein the durable hardware component includes an optical system associated with the separator.

Aspect 5. The system of Aspect 4, wherein the target blood component is separated red blood cells and the target collection parameter is a target red blood cell hematocrit.

Aspect 6. The system of Aspect 5, wherein the controller is configured to obtain the target red blood cell hematocrit by continuously adding the additive solution at a determined additive flow rate ($Q_{additive}$) to said separated red blood cells.

Aspect 7. The system of Aspect 6, wherein the controller is configured to obtain the determined additive flow rate ($Q_{additive}$) based on a flow rate of red blood cells with additive solution ($Q_{additive-RBC}$) and a flow rate of packed red blood cells exiting the separator ($Q_{RBC}$), such that:

$$Q_{additive} = Q_{additive\text{-}RBC} - Q_{RBC}.$$

Aspect 8. The system of Aspect 7, wherein the controller is further configured to determine $Q_{additive\text{-}RBC}$ based on a flow rate of pure red blood cells exiting the separator ($Q_{pure\text{-}RBC}$) and the target red blood cell product hematocrit ($H_{product\text{-}RBC}$), such that:

$$Q_{additive\text{-}RBC} = Q_{pure\text{-}RBC}/H_{product\text{-}RBC},$$

and the controller is further configured to determine $Q_{RBC}$ based on a flow rate of whole blood being pumped into the separator ($Q_{WB}$) and a flow rate of plasma exiting the separator ($Q_P$), such that:

$$Q_{RBC} = Q_{WB} - Q_P.$$

Aspect 9. The system of Aspect 8, wherein $Q_{WB}$ and $Q_P$ are pre-determined flow rates.

Aspect 10. The system of Aspect 8, wherein $Q_{WB}$ and $Q_P$ are determined by the controller based on data from the weight scales and/or the optical system.

Aspect 11. The system of any one of Aspects 8-10, wherein the controller is further configured to determine $Q_{pure\text{-}RBC}$ based on $Q_{RBC}$ and a hematocrit of packed red blood cells ($H_{RBC}$), such that:

$$Q_{pure\text{-}RBC} = Q_{RBC}*H_{RBC}.$$

Aspect 12. The system of Aspect 6, wherein the controller is configured to obtain the target red blood cell hematocrit by adding a determined volume of additive solution ($V_{additive}$) to the separated red blood cells.

Aspect 13. The system of Aspect 12, wherein the controller is configured to determine $V_{additive}$ based on a volume of pure red blood cells ($V_{pure\text{-}RBC}$), the target red blood cell product hematocrit ($H_{product\text{-}RBC}$), and a volume of packed red blood cells ($V_{RBC}$) such that:

$$V_{additive} = (V_{pure\text{-}RBC}/(H_{product\text{-}RBC})) - V_{RBC}.$$

Aspect 14. The system of Aspect 13, wherein the controller is further configured to determine $V_{pure\text{-}RBc}$ by multiplying a total amount blood to be processed by the hematocrit of the blood to be processed and $V_{RBC}$ by dividing $V_{pure\text{-}RBc}$ by the packed red blood cell hematocrit.

Aspect 15. The system of Aspect 14, wherein the packed red blood cell hematocrit is a constant empirically derived hematocrit or is determined by the controller based on data from the optical system.

Aspect 16. The system of Aspect 14, wherein the constant empirically derived hematocrit is 80%.

Aspect 17. The system of any one of Aspects 13-16, wherein $V_{additive}$ is added to the separated red blood cells after the red blood cells have been collected in the product container.

Aspect 18. The system of any one of Aspects 6-17, wherein the additive solution is Adsol.

Aspect 19. The system of Aspect 4 wherein said blood component is separated platelets.

Aspect 20. The system of Aspect 19, wherein the target collection parameter is a target concentration of platelets.

Aspect 21. The system of Aspect 19 wherein said target collection parameter is a target platelet count.

Aspect 22. The system of any one of Aspects 19-21 wherein said separator includes a spinning membrane.

Aspect 23. The system of any one of Aspects 19-22, wherein the additive solution is a platelet additive solution (PAS).

Aspect 24. The system of any one of Aspects 19-23, wherein the controller is configured to obtain a determined additive flow rate ($Q_{PAS}$) based on a flow rate of platelets with additive solution ($Q_{PAS\text{-}PLTs}$) and a flow rate of platelets ($Q_{PLTs}$), such that:

$Q_{PAS} = Q_{PAS\text{-}PLTs} - Q_{PLTs}$, and $Q_{PAS\text{-}PLTs}$ is determined by the flow rate of pure platelets and the target collection parameter, such that $Q_{PAS\text{-}PLTs} = Q_{pure\text{-}PLTs}/(\text{target collection parameter})$.

Aspect 25. A method of obtaining a separated blood component product including separating and collecting a blood component from a blood source using a system including a durable hardware component including a separator and a controller, wherein the controller is configured to automatically operate the system to separate a target blood component from a blood source, and determine and add a custom amount of additive solution to the target blood component to obtain a blood component product with a target collection parameter; and a disposable fluid flow circuit for mounting onto said durable hardware component including a separation chamber, and a plurality of containers in fluid communication with the separation chamber, wherein the plurality of containers includes at least a product container configured to contain the blood component product and an additive solution container configured to contain an additive solution; and adding the custom amount of additive solution to the blood component.

Aspect 26. The method of Aspect 25, wherein the blood source includes whole blood or a previously separated blood component.

Aspect 27. The method of Aspect 26, wherein the durable hardware component includes at least one weight scale associated with the plurality of containers.

Aspect 28. The method of Aspect 27, wherein the durable hardware component includes an optical system associated with the separator.

Aspect 29. The method of Aspect 28, wherein the target blood component is separated red blood cells and the target collection parameter is a target red blood cell hematocrit.

Aspect 30. The method of Aspect 29, including obtaining the target red blood cell hematocrit by continuously adding the custom amount of additive solution at a determined additive flow rate ($Q_{additive}$) to the separated red blood cells.

Aspect 31. The method of Aspect 30, wherein the controller is configured to obtain the determined additive flow rate ($Q_{additive}$) based on a flow rate of red blood cells with additive solution ($Q_{additive-RBC}$) and a flow rate of packed red blood cells exiting the separator ($Q_{RBC}$), such that:

$$Q_{additive} = Q_{additive-rbc} - Q_{RBC}.$$

Aspect 32. The method of Aspect 31, wherein the controller is further configured to determine $Q_{additive-RBC}$ based on a flow rate of pure red blood cells exiting the separator ($Q_{pure-RBC}$) and the target red blood cell product hematocrit ($H_{product-RBC}$), such that:

$$Q_{additive-RBC} = Q_{pure-RBC}/(H_{product-RBC}),$$

and the controller is further configured to determine $Q_{RBC}$ based on a flow rate of whole blood being pumped into the separator ($Q_{WB}$) and a flow rate of plasma exiting the separator ($Q_P$), such that:

$$Q_{RBC} = Q_{WB} - Q_P.$$

Aspect 33. The method of Aspect 32, wherein $Q_{WB}$ and $Q_P$ are pre-determined flow rates.

Aspect 34. The method of Aspect 32, wherein $Q_{WB}$ and $Q_P$ are determined by the controller based on data from the weight scales and/or the optical system.

Aspect 35. The method of any one of Aspects 32-34, wherein the controller is further configured to determine $Q_{pure-RBC}$ based on $Q_{RBC}$ and a hematocrit of packed red blood cells ($H_{RBC}$), such that:

$$Q_{pure-RBC} = Q_{RBC}*H_{RBC}.$$

Aspect 36. The method of Aspect 28, including obtaining the target red blood cell hematocrit by adding the custom amount of additive solution at a determined volume of additive solution ($V_{additive}$) to the separated red blood cells.

Aspect 37. The method of Aspect 36, wherein the controller is configured to determine $V_{additive}$ based on a volume of pure red blood cells ($V_{pure-RBC}$), the target red blood cell product hematocrit ($H_{product-RBC}$), and a volume of packed red blood cells ($V_{RBC}$) such that:

$$V_{additive} = (V_{pure-RBC}/(H_{product-RBC})) - V_{RBC}.$$

Aspect 38. The method of Aspect 37, wherein the controller is further configured to determine $V_{pure-RBc}$ by multiplying a total amount blood to be processed by the hematocrit of the blood source and $V_{RBC}$ by dividing $V_{pure-RBc}$ by the packed red blood cell hematocrit.

Aspect 39. The method of Aspect 38, wherein the packed red blood cell hematocrit is a constant empirically derived hematocrit or is determined by the controller based on data from the optical system.

Aspect 40. The method of Aspect 39, wherein the constant empirically derived hematocrit is 80%.

Aspect 41. The method of any one of Aspects 36-40, wherein $V_{additive}$ is added to the separated red blood cells after the red blood cells have been collected in the product container.

Aspect 42. The method of any one of Aspects 29-41, wherein the additive solution is Adsol.

Aspect 43. The method of Aspect 28, wherein the blood component is separated platelets.

Aspect 44. The method of Aspect 43, wherein the target collection parameter is a target concentration of platelets.

Aspect 45. The method of Aspect 43, wherein the target collection parameter is a target platelet count.

Aspect 46. The method of any one of Aspects 43-45, wherein the separator includes a spinning membrane.

Aspect 47. The method of any one of Aspects 43-46, wherein the additive solution is a platelet additive solution (PAS).

Aspect 48. The method of any one of Aspects 43-47, wherein the controller is configured to obtain a determined additive flow rate ($Q_{PAS}$) based on a flow rate of platelets with additive solution ($Q_{PAS-PLTs}$) and a flow rate of platelets ($Q_{PLTs}$), such that: $Q_{PAS} = Q_{PAS-PLTs} - Q_{PLTs}$, and $Q_{PAS-PLTs}$ is determined by the flow rate of pure platelets and the target collection parameter, such that $Q_{PAS-PLTs} = Q_{pure-PLTs}$/(target collection parameter).

Aspect 49. A blood processing device for collecting a separated blood component product including a separator; and a controller, wherein the controller is configured to automatically operate the device to separate a target blood component from a blood source, and determine and add a custom amount of additive solution to the target blood component to obtain a blood component product with a target collection parameter; and wherein the device is configured to be associated with a disposable fluid circuit.

Aspect 50. The device of Aspect 49, wherein the blood source includes whole blood or a previously separated blood component.

Aspect 51. The device of Aspect 50, including at least one weight scale.

Aspect 52. The device of Claim 51, including an optical system associated with the separator.

Aspect 53. The device of Aspect 52, wherein the controller is configured to separate red blood cells from the blood source and obtain a red blood cell product with a target red blood cell hematocrit.

Aspect 54. The device of Aspect 53, wherein the controller is configured to obtain the target red blood cell hematocrit by continuously adding the additive solution at a determined additive flow rate ($Q_{additive}$) to said separated red blood cells.

Aspect 55. The device of Aspect 54, wherein the controller is configured to obtain the determined additive flow rate ($Q_{additive}$) based on a flow rate of red blood cells with additive solution ($Q_{additive-RBC}$) and a flow rate of packed red blood cells exiting the separator ($Q_{RBC}$), such that:

$$Q_{additive} = Q_{additive-RBC} - Q_{RBC}.$$

Aspect 56. The device of Aspect 55, wherein the controller is further configured to determine $Q_{additive-RBC}$ based on a flow rate of pure red blood cells exiting the separator ($Q_{pure-RBC}$) and the target red blood cell product hematocrit ($H_{product-RBC}$), such that:

$$Q_{additive-RBC} = Q_{pure-RBC}/H_{product-RBC},$$

and the controller is further configured to determine $Q_{RBC}$ based on a flow rate of whole blood being pumped into the separator ($Q_{WB}$) and a flow rate of plasma exiting the separator ($Q_P$), such that:

$$Q_{RBC} = Q_{WB} - Q_P.$$

Aspect 57. The device of Aspect 56, wherein $Q_{WB}$ and $Q_P$ are pre-determined flow rates.

Aspect 58. The device of Aspect 56, wherein $Q_{WB}$ and $Q_P$ are determined by the controller based on data from the weight scales and/or the optical system.

Aspect 59. The device of any one of Aspects 56-58, wherein the controller is further configured to determine $Q_{pure-RBC}$ based on $Q_{RBC}$ and a hematocrit of packed red blood cells ($H_{RBC}$), such that:

$$Q_{pure-RBC} = Q_{RBC}*H_{RBC}.$$

Aspect 60. The device of Aspect 54, wherein the controller is configured to obtain the target red blood cell hematocrit by adding a determined volume of additive solution ($V_{additive}$) to the separated red blood cells.

Aspect 61. The device of Aspect 60, wherein the controller is configured to determine $V_{additive}$ based on a volume of pure red blood cells ($V_{pure-RBC}$), the target red blood cell product hematocrit ($H_{product-RBC}$), and a volume of packed red blood cells ($V_{RBC}$) such that:

$$V_{additive} = (V_{pure-RBC}/(H_{product-RBC})) - V_{RBC}.$$

Aspect 62. The device of Aspect 61, wherein the controller is further configured to determine $V_{pure\text{-}RBc}$ by multiplying a total amount blood to be processed by the hematocrit of the blood source and $V_{RBC}$ by dividing $V_{pure\text{-}RBc}$ by the packed red blood cell hematocrit.

Aspect 63. The device of Aspect 62, wherein the packed red blood cell hematocrit is a constant empirically derived hematocrit or is determined by the controller based on data from the optical system.

Aspect 64. The device of Aspect 63, wherein the constant empirically derived hematocrit is 80%.

Aspect 65. The device of any one of Aspects 60-64, wherein $V_{additive}$ is added to the separated red blood cells after the red blood cells have been collected in a product container associated with the disposable fluid circuit.

Aspect 66. The device of any one of Aspects 53-65 wherein the additive solution is Adsol.

Aspect 67. The device of Aspect 52, wherein the controller is configured to separate platelets from the blood source and obtain a platelet product with a target collection parameter.

Aspect 68. The device of Aspect 67, wherein the target collection parameter is a target concentration of platelets.

Aspect 69. The device of Aspect 68, wherein the target collection parameter is a target platelet count.

Aspect 70. The device of any one of Aspects 67-69 wherein said separator includes a spinning membrane.

Aspect 71. The device of any one of Aspects 67-70, wherein the additive solution is a platelet additive solution (PAS).

Aspect 72. The device of any one of Aspects 67-71, wherein the controller is configured to obtain a determined additive flow rate ($Q_{PAS}$) based on a flow rate of platelets with additive solution ($Q_{PAS\text{-}PLTs}$) and a flow rate of platelets ($Q_{PLTs}$), such that: $Q_{PAS} = Q_{PAS\text{-}PLTs} - Q_{PLTs}$, and QPAS-PLTS is determined by the flow rate of pure platelets and the target collection parameter, such that $Q_{PAS\text{-}PLTs} = Q_{pure\text{-}PLTs}/(\text{target collection parameter})$.

It will be understood that the embodiments and examples described above are illustrative of some of the applications or principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that the claims may be directed to the features thereof, including as combinations of features that are individually disclosed or claimed herein.

**Claims**

1. A system for collecting a blood component comprising:

   a durable hardware component comprising:

   a separator; and
   a controller, wherein the controller is configured to:

   automatically operate the system to separate a target blood component from a blood source, and determine and add a custom amount of additive solution to the target blood component to obtain a blood component product

   with a target collection parameter; and

   a disposable fluid flow circuit for mounting onto said durable hardware component comprising:

   a separation chamber, and
   a plurality of containers in fluid communication with the separation chamber, wherein the plurality of containers includes at least a product container configured to contain the blood component product and an additive solution container configured to contain an additive solution.

2. The system of Claim 1, wherein the blood source comprises whole blood or a previously separated blood component, and wherein the durable hardware component includes at least one weight scale associated with the plurality of containers and an optical system associated with the separator.

3. The system of Claim 2, wherein the target blood component is separated red blood cells, and the target collection parameter is a target red blood cell product hematocrit.

4. The system of Claim 3, wherein the controller is configured to obtain the target red blood cell product hematocrit by continuously adding the additive solution at a determined additive flow rate ($Q_{additive}$) to said separated red blood cells.

5. The system of Claim 4, wherein the controller is configured to obtain the determined additive flow rate ($Q_{additive}$) based on a flow rate of red blood cells with additive solution ($Q_{additive-RBC}$) and a flow rate of packed red blood cells exiting the separator ($Q_{RBC}$), such that:

$$Q_{additive} = Q_{additive-RBC} - Q_{RBC}.$$

6. The system of Claim 5, wherein the controller is further configured to determine $Q_{additive-RBC}$ based on a flow rate of pure red blood cells exiting the separator ($Q_{pure-RBC}$) and the target red blood cell product hematocrit ($H_{product-RBC}$), such that:

$$Q_{additive-RBC} = Q_{pure-RBC}/H_{product-RBC},$$

and the controller is further configured to determine $Q_{RBC}$ based on a flow rate of whole blood being pumped into the separator ($Q_{WB}$) and a flow rate of plasma exiting the separator ($Q_P$), such that:

$$Q_{RBC} = Q_{WB} - Q_P,$$

and wherein $Q_{WB}$ and $Q_P$ are pre-determined flow rates or are determined by the controller based on data from the weight scales and/or the optical system.

7. The system of Claim 6, wherein the controller is further configured to determine $Q_{pure-RBC}$ based on $Q_{RBC}$ and a hematocrit of packed red blood cells ($H_{RBC}$), such that:

$$Q_{pure-RBC} = Q_{RBC}*H_{RBC}.$$

8. The system of Claim 4, wherein the controller is configured to obtain the target red blood cell product hematocrit by adding a determined volume of additive solution ($V_{additive}$) to the separated red blood cells.

9. The system of Claim 8, wherein the controller is configured to determine $V_{additive}$ based on a volume of pure red blood cells ($V_{pure-RBC}$), the target red blood cell product hematocrit ($H_{product-RBC}$), and a volume of packed red blood cells ($V_{RBC}$) such that:

$$V_{additive} = (V_{pure-RBC}/(H_{product-RBC})) - V_{RBC}.$$

10. The system of Claim 9, wherein the controller is further configured to determine $V_{pure-RBc}$ by multiplying a total amount blood to be processed by the hematocrit of the blood to be processed and $V_{RBC}$ by dividing $V_{pure-RBc}$ by a packed red blood cell hematocrit, and wherein the packed red blood cell hematocrit is a constant empirically derived hematocrit or is determined by the controller based on data from the optical system.

11. The system of any one of Claims 9-10, wherein $V_{additive}$ is added to the separated red blood cells after the red blood cells have been collected in the product container.

12. The system of Claim 2 wherein said target blood component is separated platelets, and the target collection parameter is a target concentration of platelets or a target platelet count.

13. The system of Claim 12, wherein the controller is configured to obtain a determined additive flow rate ($Q_{PAS}$) based on a flow rate of platelets with additive solution ($Q_{RAS-RLTs}$) and a flow rate of platelets ($Q_{PLTs}$), such that: $Q_{PAS} = Q_{PAS-PLTs} - Q_{PLTs}$, and $Q_{PAS-PLTs}$ is determined by the flow rate of pure platelets and the target collection parameter, such that $Q_{PAS-PLTs} = Q_{pure-PLTs}/(\text{target collection parameter})$.

14. A method of obtaining a separated blood component product comprising:
separating and collecting a blood component from a blood source using a system comprising:

a durable hardware component comprising:

a separator; and
a controller, wherein the controller is configured to:

automatically operate the system to separate a target blood component from a blood source, and determine and add a custom amount of additive solution to the target blood component to obtain a blood component product with a target collection parameter; and

a disposable fluid flow circuit for mounting onto said durable hardware component comprising:

a separation chamber, and
a plurality of containers in fluid communication with the separation chamber, wherein the plurality of containers includes at least a product container configured to contain the blood component product and an additive solution container configured to contain an additive solution; and adding the custom amount of additive solution to the blood component.

15. A blood processing device for collecting a separated blood component product comprising:

a separator; and
a controller, wherein the controller is configured to:

automatically operate the device to separate a target blood component from a blood source, and determine and add a custom amount of additive solution to the target blood component to obtain a blood component product with a target collection parameter; and

wherein the device is configured to be associated with a disposable fluid circuit.

**FIG. 1**

**FIG. 2**

**FIG. 3**

**FIG. 4**

Europäisches
Patentamt

European
Patent Office

Office européen
des brevets

# EUROPEAN SEARCH REPORT

Application Number

EP 24 19 3591

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/194824 A1 (FENWAL INC [US]) 30 September 2021 (2021-09-30) * target amount of additive solution; page 24, line 5 - line 9; claim 7; figures * | 1-15 | INV. A61M1/02 A61M1/36 |
| A | US 2022/409780 A1 (REBULLA PAOLO [IT] ET AL) 29 December 2022 (2022-12-29) * paragraph [0023] * | 1,14 | |

TECHNICAL FIELDS
SEARCHED       (IPC)

A61M

The present search report has been drawn up for all claims

| Place of search | Date of completion of the search | Examiner |
|---|---|---|
| The Hague | 15 November 2024 | Lakkis, Angeliki |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**EP 4 506 022 A1**

## ANNEX TO THE EUROPEAN SEARCH REPORT
## ON EUROPEAN PATENT APPLICATION NO.

EP 24 19 3591

15-11-2024

| Patent document cited in search report | Publication date | Patent family member(s) | | Publication date |
|---|---|---|---|---|
| WO 2021194824 A1 | 30-09-2021 | EP | 4126096 A1 | 08-02-2023 |
| | | JP | 2023518823 A | 08-05-2023 |
| | | US | 2024226396 A1 | 11-07-2024 |
| | | WO | 2021194824 A1 | 30-09-2021 |
| US 2022409780 A1 | 29-12-2022 | AU | 2020387128 A1 | 09-06-2022 |
| | | CA | 3158710 A1 | 27-05-2021 |
| | | EP | 4061444 A1 | 28-09-2022 |
| | | IT | 201900021426 A1 | 18-05-2021 |
| | | JP | 2023502469 A | 24-01-2023 |
| | | US | 2022409780 A1 | 29-12-2022 |
| | | WO | 2021099953 A1 | 27-05-2021 |

EPO FORM P0459

**EP 4 506 022 A1**

**Patent documents cited in the description**

- WO 2021194824 A
- US 8075468 B
- US 4157723 A
- US 4753697 A
- US 5158630 A
- US 5156701 A
- US 10307582 B
- US 10040247 B
- US 9440396 B
- US 20190201916
- US 6849039 B
- US 6899666 B
- US 20180078582
- US 9402866 B
- US 11465169 B